**Europäisches Patentamt**

**European Patent Office**

**Office européen des brevets**

(19)

(11) Publication number : **0 501 752 A1**

(12) # EUROPEAN PATENT APPLICATION

(21) Application number : **92301576.2**

(22) Date of filing : **25.02.92**

(51) Int. Cl.⁵ : **A61L 9/22, A61L 9/18**

(30) Priority : **28.02.91 MX 24731**

(43) Date of publication of application :
**02.09.92 Bulletin 92/36**

(84) Designated Contracting States :
**AT BE CH DE DK ES FR GB GR IT LI LU NL PT SE**

(71) Applicant : **Martinez-Lara, Estanislao**
**Ave. C. Junco de la Vega 208, Col. Roma 64700**
**Monterrey, Nuevo Leon (MX)**

(72) Inventor : **Martinez-Lara, Estanislao**
**Ave. C. Junco de la Vega 208, Col. Roma 64700**
**Monterrey, Nuevo Leon (MX)**

(74) Representative : **Atkinson, Peter Birch**
**Marks & Clerk Suite 301 Sunlight House Quay Street**
**Manchester M3 3JY (GB)**

(54) **Multiple piramidal body to sterilize oxygenate and deodorize the atmosphere.**

(57)    This invention refers to an apparatus and its mechanism. More specifically to a mostly pyramidal apparatus that has a combination of convergent walls in four planes, each one of them being a resultant of one side of the pyramid which has four equilateral triangles, and these are inside a square box. Inside the square box we find the complement of the apparatus, that is the reactors, transformers and circuits which execute the pyramid's operation. This apparatus also has mirrors and cristals in combination with non ferric metal alloy sheets to form the pyramid.

FIG 1

EP 0 501 752 A1

Jouve, 18, rue Saint-Denis, 75001 PARIS

TO THE COMMISSIONER OF PATENTS:

Your petitioner ESTANISLAO MARTINEZ-LARA, a Mexican citizen, resident of Monterrey, Nuevo Leon, Mexico, whose post office address is Ave. Junco de la Vega #208 Colonia Roma 64700, Monterrey, Nuevo Leon, Mexico, prays that letter patent may be granted to him for MULTIPLE PIRAMIDAL BODY TO STERILIZE OXYGENATE AND DEODORIZE THE ARMOSPHERE.

## SUMMARY OF THE INVENTION

This invention refers to an apparatus and its mechanism. More specifically to a mostly pyramidal apparatus that has a combination of convergent walls in four planes, each one of them being a resultant of one side of the pyramid which has four equilateral triangles, and these are inside a square box. Inside the square box we find the complement of the apparatus, that is the reactors, transformers and circuits which execute the pyramid's operation. This apparatus also has mirrors and cristals in combination with non ferric metal alloy sheets to form the pyramid.

## BACKGROUND OF THE INVENTION

Man, since his very first days on earth has always tried to use the resources provided by nature to improve his own life and the environment. Back then the egyptians used the pyramid shape fully trusting that its shape provided a concentration of elements existing in nature and provided the means to strengthen the healt and the environment which in turn reinforced the health. Through time there have been several mechanisms and inventions to try to take advantage of the benefits of the pyramid for their own daily use. However, until now there were. so many difficulties to build a mechanism with the previously mentioned advantages which at the same time would be easy to operate and move seeking to provide the advantages of the pyramid shape in different places, even thou they were geographically separated.

With this invention, for the first time in human history, the party I represent has been able to unite all the elements which favor the action and the effects of the pyramid in a device fully transportable and easily placed not only on the outside but also inside buildings, homes or shops, which at a given moment will receive the benefits of the operation of the pyramid, object of this invention.

As we go through the description of the invention it will be easier to understand some other concepts, and for that reason the party I represent has no intention to restrict the reach of his invention just to the description and the drawings in the following pages. On the contrary, he claims any and all varieties, which with the before described principles, that have the same effects.

## BRIEF DESCRIPTION OF THE DRAWINGS

Figure #1, shows a conventional view of the pyramid.

Figure #2, shows a top view of the pyramid.

Figure #3, shows a raised view of the section A-A 1.

Figure #4, shows a raised view of the section B-B 1.

Figure #5, shows an electrical diaphragm of the pyramid's circuit.

## DETAILED DESCRIPTION OF THE INVENTION

In reference to said figures, this pyramidal device is made up of a solid wood box (1), which has legs (2) allowing it to be above the floor; on the wooden (1) box there are several major (3) pyramids and a central pyramidal (4B) body formed by alternate sheets of crystal - mirror and metal sheets (5) with a non ferric alloy, in such a way that on top of the combination of sheets we find a crystal (5) sheet and immediately afterwards we find a non ferric (5) metal sheet, and in this sequence, the four elements or triangular bodies (3) converging from its base towards its center, thereby forming an equilateral triangle with the four bodies or figures.

In order to hold the sheets in place, the same are firmly supported by a sandwich bar (6) and the base (4A) of the crystal sheets (4) enters the sandwich bar, meanwhile the non ferric metal alloy metal sheets (5) are held in a central position between the crystal sheet (4); the sandwich bar (6) is firmly mounted on the four walls of the box (1) and the sandwich bar (6) has a peripheral displacement, that is, it slides along the inner part (1A) of the box's walls (1).

Inside the box, in the center of the box (1) we find a reflecting mirror (7) placed horizontally and laying on top of a sponge (8) base, thereby providing a certain degree of shock absorption, but at the same time it is entirely stable, the center of the reflecting mirror (7A) lies exactly in the center of the converging (27) point of the four triangular bodies (3). Under these conditions the mirror (7) reflects in. equally symetric planes the corresponding portions of the crystal sheets (4) and the metal sheets (5) of a non ferric alloy.

Exactly under the reflecting mirror (7) and under the sponge (8) base we find an alternating current (9) transformer which receives a specific voltage and then increases the same in a very high proportion, in such a way that the resulting voltage is infinitely greater than the incoming voltage.

Placed exactly under the transformer (9) and duly held in place to the chasis (10), we find a fan (11) which is used to expel the air from inside the box (1) to the outside, and said air shall have to come in contact throughout the pyramid with the crystal - mirror sheets (4) and the non metal sheets (5). Under the fan we find a cone (17) which helps send the air from the fan (11) towards the upper part of the box (1), and as a result the air passes through the upper pyramidal body; the chasis (10) is firmly mounted inside the box (1) by means of jambs (12) to which it is united by fasteners (14) being either screws or bolts, we also find coils (15) inside the box (1) and these coils are placed on supports (16) and interconnected to the pyramid's electrical circuit.

Also inside the box (1) we find the control box (18) which houses elements such as the buttons (19) and the panel (25), making the circuit's operation and the pyramid's operation feasible.

The pyramid's electrical circuit has, besides the fan (11), the transformer (9) the coils (15) and the controls (19), a plurality of horizontal plates (20) which all together provide the possibility for the transformer (9) to change the low incoming voltage into a higher voltage. The electrical circuit also has a selecting mechanism (21) through which it is possible to increase or decrease the power or the voltage provided by the transformer (9), that is, it can go from 0 volts to the maximum allowed by the transformer (9), and in the same circuit we find interruptors (22) to turn the electrical circuit on or off, fuses (23) and a pilot (24) to check if the machine is on or off.

The panel (25) is visible on the outside of the box (1) and of course the different controls (19) which allow the circuit to function.

Also, to facilitate its handling, there are some holes (26) on two of the walls of the box (1) in order to make it easier to move the pyramid to any other place.

OPERATING METHOD

In order to operate the pyramid and receive the results for which this invention has been built it can be placed either outside or inside, connect an electrical extension cord, turn it on and adjust the power according to the area to be influenced by the pyramid's operation, thereby achieving purification and the before mentioned advantages.

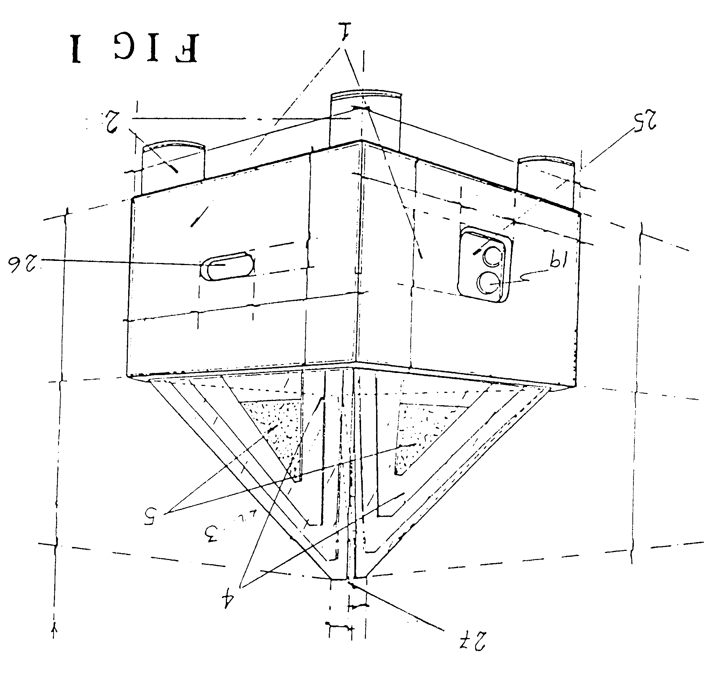

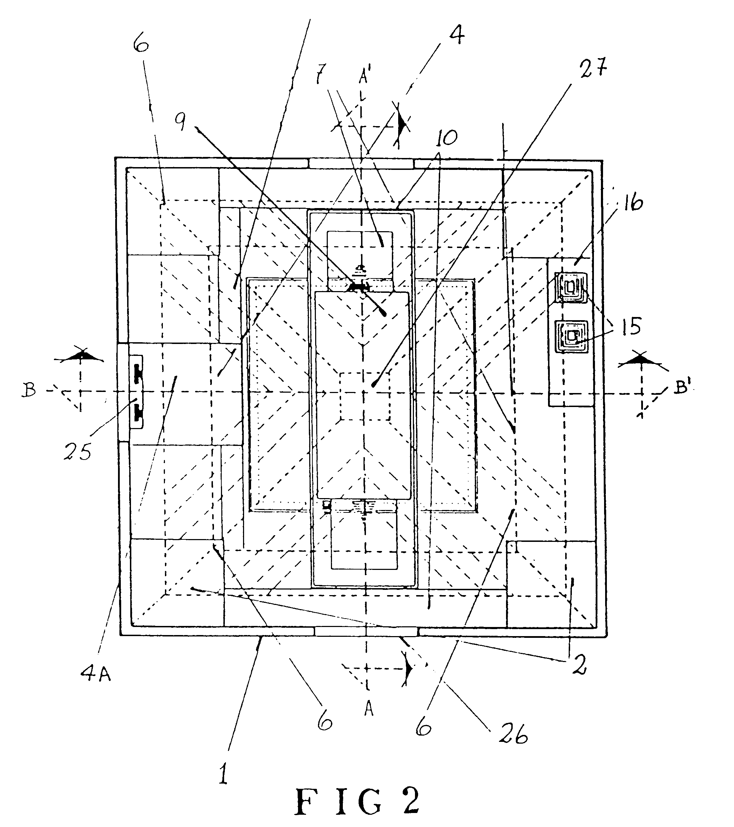

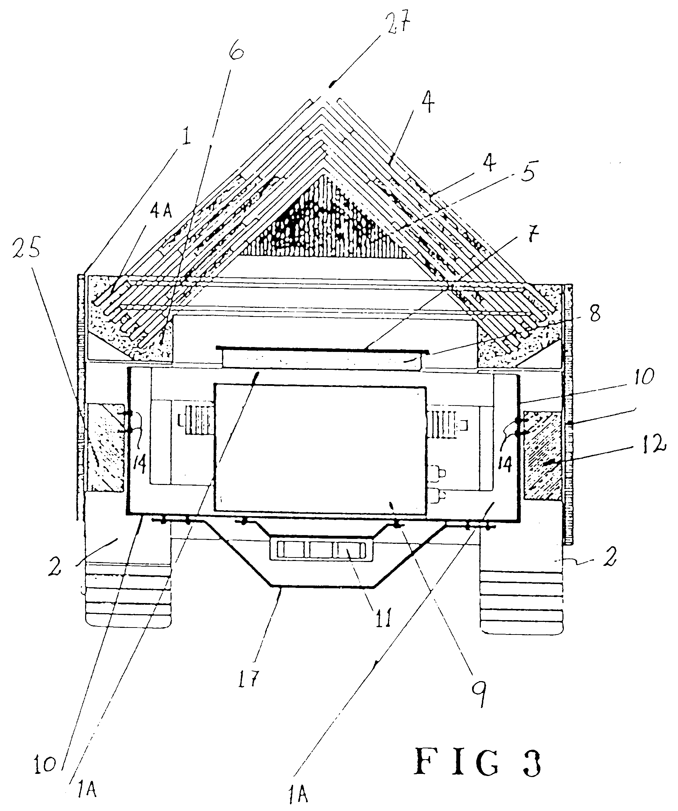

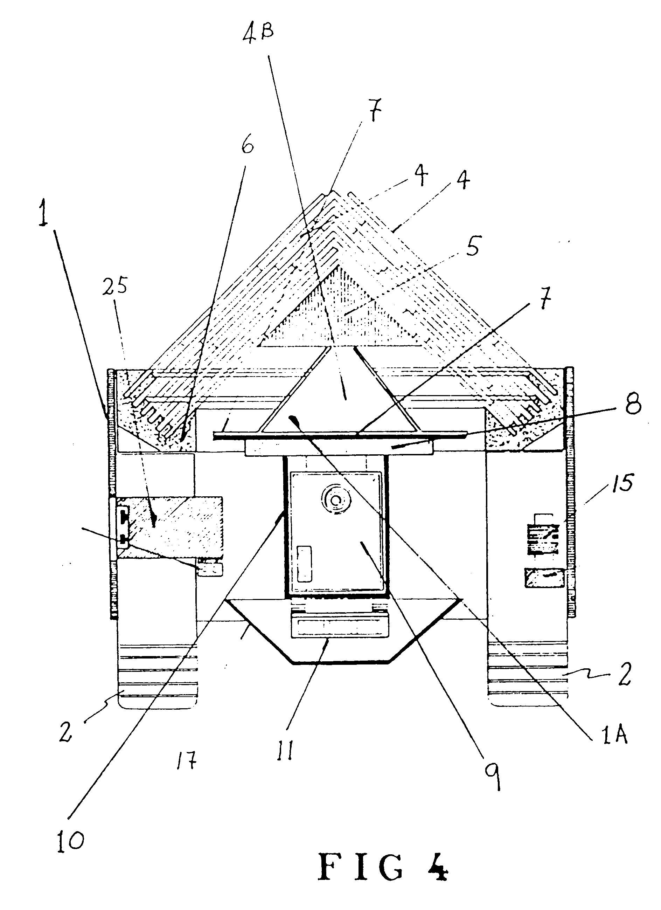

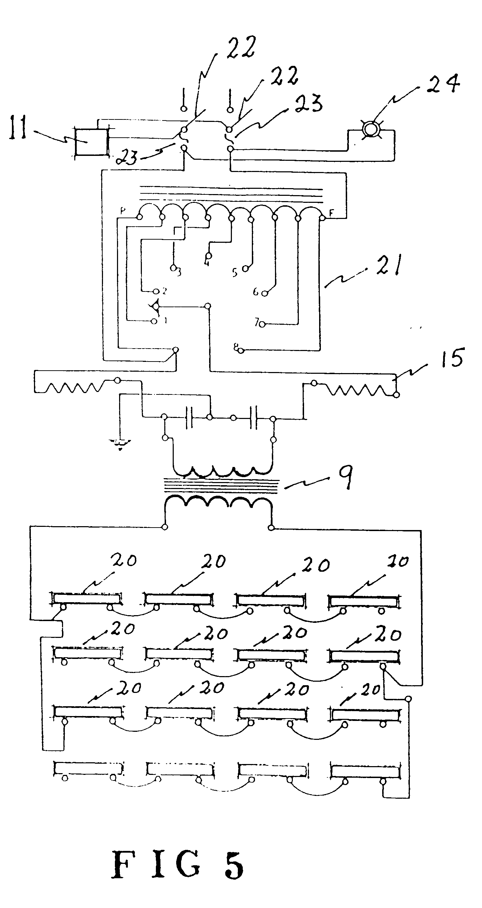

**Claims**

1. A multiple pyramidal body to sterilize, oxygenate and deodorize the environment and more specifically a pyramid characterized by a box having legs (or other support means) separated from the floor, and inside, one on top of the other, a plurality of pyramidal bodies; mirror-crystal sheets alternated with a non-ferric alloy in the sequence first a crystal sheet, next a non-ferric alloy metal sheet and with this sequence the four triangular bodies converge from their base to the center, thereby forming an equilateral pyramid; and a central pyramidal body placed under the before mentioned bodies with its apex having the same convergence as the other pyramids.

2. A multiple pyramidal body to sterilize, oxygenate and deodorize the environment, as claimed in claim I characterized by a support or a sandwich bar at its internal periphery, into which every crystal sheet is located, the non-ferric metal alloy sheets being set between the crystal plates to form a sandwich; a reflecting mirror placed horizontally inside the box and supported on a sponge-type base which provides a degree of shock absorption whilst remaining in the same position; the convergence center resulting from the convergence of the pyramid's walls coinciding with the center of the reflecting mirror so that the crystal sheets and the non-ferric alloy metal sheets are reflected in equally symmetrical planes converging towards the convergence center.

3. A multiple pyramidal body to sterilize, oxygenate and deodorize the environment, as claimed in claim I or claim 2, characterized by an alternating current transformer placed inside the pyramid; a plurality of high voltage horizontal sheets installed at the secondary to vary the voltage; a fan under the transformer to expel the air from inside the box to the outside, such that when the air leaves the pyramid it comes in contact with the voltage transformer and the pyramid's walls; and a cone under the fan to direct the air to the higher pyramidal bodies.

4. A multiple pyramidal body to sterilize, oxygenate and deodorize the environment, as claimed in any preceding claim, characterized by coils which placed on their supports are interconnected to the pyramid's electrical circuit in order to operate synchronically with the fan and the transformer; a chassis mounted inside the box by means. of girders to provide the necessary supports to place the different parts of the pyramidal body.

5. A multiple pyramidal body to sterilize, oxygenate and deodorize the environment as claimed in any preceding claim, characterized by a control box to operate and monitor the operation of the pyramidal body; and an electrical circuit with power lines, fuses and interruptors to operate the transformer.

6. Air purification apparatus comprising air circulation means, an air outlet and an air purification assembly, characterized in that the air purification assembly is of pyramidal shape, at least one wall of which comprises a layer of a crystal material and a layer of a non-ferrous metal alloy.

FIG 1

F I G 2

F I G 3

FIG 4

F I G 5

**European Patent Office**

## EUROPEAN SEARCH REPORT

Application Number

EP    92 30 1576

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | DE-A-3 023 991 (H. HANSCHMANN) <br> --- | | A61L9/22 <br> A61L9/18 |
| A | US-A-4 391 773 (G. P. FLANAGAN) <br> --- | | |
| A | FR-A-2 189 087 (DUMONT ENGINEERING) <br><br> ----- | | |
| | | | **TECHNICAL FIELDS SEARCHED (Int. Cl.5 )** <br><br> A61L |

The present search report has been drawn up for all claims

| Place of search | Date of completion of the search | Examiner |
|---|---|---|
| THE HAGUE | 18 JUNE 1992 | PELTRE CHR. |

EPO FORM 1503 03.82 (P0401)